Europäisches Patentamt

European Patent Office

Office européen des brevets

(11) Veröffentlichungsnummer: **0 155 933**

A2

(12) **EUROPÄISCHE PATENTANMELDUNG**

(21) Anmeldenummer: 85890074.9

(22) Anmeldetag: 21.03.85

(51) Int. Cl.⁴: **A 61 L 2/06**
**A 61 L 2/24**

(30) Priorität: 23.03.84 AT 988/84

(43) Veröffentlichungstag der Anmeldung:
25.09.85 Patentblatt 85/39

(84) Benannte Vertragsstaaten:
DE GB IT SE

(71) Anmelder: VEREINIGTE EDELSTAHLWERKE
AKTIENGESELLSCHAFT (VEW)
Elisabethstrasse 12
A-1010 Wien(AT)

(72) Erfinder: Macho, Winfried, Dipl.-Ing.
Koflergasse 9/16
A-1120 Wien(AT)

(72) Erfinder: Miksitz, Franz
Agnesgasse 18
A-2630 Ternitz(AT)

(74) Vertreter: Widtmann, Georg, Dr.
Vereinigte Edelstahlwerke Aktiengesellschaft (VEW)
Elisabethstrasse 12
A-1010 Wien(AT)

(54) Dampfsterilisator.

(57) Die Erfindung betrifft einen Dampfsterilisator mit zumindest einem druckdicht abschließbaren Sterilisationsraum, in den das zu sterilisierende Gut, insbesondere Flüssigkeit enthaltende Behälter, z.B. Flaschen, Beutel od. dgl., einbringbar ist, welcher Dampfzuleitungen und Gas- und/oder Flüssigkeitsableitungen aufweist, wobei eine Steuerung mit Temperaturmeßfühler im Sterilisationsraum vorgesehen ist, wobei der/die Meßfühler, insbesondere Temperaturmeßfühler, mit einem Sender (1) zurdrahtlosen Übertragung von Signalen, mit Antenne (9) verbunden ist/sind, der im Sterilisationsraum anordenbar ist und ein Empfänger zum Empfang der Signale außerhalb des Sterilisationsraumes angeordnet ist, welcher mit einer Antenne innerhalb desselben zur Weiterleitung der Signale an die Steuerung verbunden ist.

Fig. 1

EP 0 155 933 A2

- 1 -

Dampfsterilisator

Die Erfindung bezieht sich auf einen Dampfsterilisator mit zumindest einem druckdicht abschließbaren Sterilisationsraum.

Obwohl die Dampfsterilisation eine der ältesten Sterilisationsarten ist, hat sie sich gegenüber den neueren Methoden zur Sterilisation - sei es z.B. Gassterilisation oder über Gammastrahlen bzw. Elektronenbeschleunigern - noch immer als besonders wirtschaftlich und sicher erwiesen. Bei der Sterilisation von Flüssigkeiten muß die Zeit mitberücksichtigt werden, welche bei Erreichen der erwünschten Außentemperatur noch verstreicht, bis das zu sterilisierende Gut ebenfalls die erwünschte Höchsttemperatur erreicht hat. Die Steuerung des Sterilisators kann hiebei über Simulationsflaschen erfolgen, also einem Standardbehälter, in welchem eine Flüssigkeit angeordnet ist, in die beispielsweise ein Temperatur- und/oder Druckmeßfühler taucht. Oder es kann auch ein träger bzw. empfindlicher Temperaturmeßfühler angeordnet werden, welcher über ein eigenes Rechnerprogramm die wahrscheinliche Temperatur im zu sterilisierenden Gut errechnet, wobei die Steuerung dann über diese Werte und die Zeit durchgeführt wird.

Wesentlich für eine exakte Steuerung ist, daß die Temperatur - sei sie auf welche Art und Weise immer - über Meßfühler gemessen wird, die in unmittelbarer Nähe des zu sterilisierenden Gutes angeordnet werden. Hierfür ist es bekannt, einerseits in der Wandung des Sterilisators eine Ausnehmung vorzusehen und dort eine eigene Meßflasche od.dgl. anzuordnen. In diesem Fall kann die elektrische Leitung direkt durch die Wandung hindurch zur Steuerung gelegt werden. Es bedarf hier besonders hoher Erfahrung der Konstrukteure und auch besonders guter Bedienungsanleitungen, um eine Übereinstimmung der gemessenen
Temperaturen und der Temperatur im Sterilisationsgut
in Abhängigkeit von der Größe des zu sterilisierenden
Gutes zu erhalten, wenn besonders energiesparend, d.h.
mit geringem Dampfverbrauch, gearbeitet werden soll.

Eine weitere Anordnung der Meßfühler besteht darin,
daß dieselben direkt zwischen dem zu sterilisierenden
Gut angeordnet werden, wobei dann die elektrischen
Leitungen der Meßfühler aus dem Gut heraus, welches in
der Regel auf einem Wagen, der in den Sterilisator gefahren werden kann, angeordnet ist, durch die Wandung
hindurch zur Steuerung geführt werden müssen. Das heißt
vor und nach Sterilisation muß der Meßfühler im Wagen
angeordnet bzw. aus dem Wagen entfernt werden. Daß hiebei erhebliche Störungen auftreten, ist wohl selbstverständlich, sodaß diese Anordnung von Meßfühlern, obwohl
die Höchstgenauigkeit dadurch erreichbar wäre, in die
Praxis keinen Eingang finden konnte.

Der erfindungsgemäße Dampfsterilisator mit zumindest
einem druckdicht abschließbaren Sterilisationsraum, in
den das zu sterilisierende Gut, insbesondere Flüssigkeit enthaltende Behälter, z.B. Flaschen, Beutel od.
dgl. einbringbar ist, welcher Dampfzuleitungen und Gas-
und/oder Flüssigkeitsableitungen aufweist, wobei eine
Steuerung mit Temperaturmeßfühler im Sterilisationsraum vorgesehen ist, besteht im wesentlichen darin,
daß der/die Temperaturmeßfühler mit einem Sender zur
drahtlosen Übertragung von Signalen mit Antenne verbunden ist/sind, der im Sterilisationsraum angeordnet ist,
und ein Empfänger zum Empfang der Signale außerhalb
des Sterilisationsraumes angeordnet ist, welcher mit einer Antenne innerhalb desselben zur Weiterleitung der Signale an die Steuerung verbunden ist. Bei einem derartigen Dampfsterilisator ist die drahtlose Übertragung der Meßdaten, die direkt in unmittelbarer Nähe des zu sterilisierenden Gutes aufgenommen werden können, an die Steuerung möglich, wobei durch diese spezifische Anordnung der Antennen mit geringer Energie das Auslangen gefunden werden kann, wodurch lediglich geringster Platzbedarf gegeben ist. Ist die Antenne des Empfängers gegenüber den Wänden des Sterilisationsraumes elektrisch isoliert, so können Störsignale od. dgl. besonders leicht vermieden werden, wobei gleichzeitig Energieverluste der Signale, z.B. durch geerdete Wandung oder zu große Masse der Wandung vermieden wird.

Der Sender kann in einem, insbesondere metallischen, wasserdampfdichten Behälter angeordnet sein, wodurch ein Sender offener Bauart eingesetzt werden kann. Ist der Sender in einem evakuierten Behälter angeordnet, so kann der Sender im Sterilisator ohne Temperaturbeeinträchtigung über längere Zeit in Betrieb gehalten werden. Vorteilhafterweise weist der Behälter einen Doppelmantel auf, welcher evakuiert ist, wobei gegebenenfalls zur weiteren Aufrechterhaltung eines Vakuums ein feinteiliger Füllstoff in demselben angeordnet sein kann.

Ist der Sender in einem Feststoff mit einem Schmelzpunkt zwischen 30 und 70°C, insbesondere zwischen 40 und 60°C eingeschmolzen, so kann bei Erwärmung der Sonde lange die Temperatur auf den Schmelzpunkt des Feststoffes gehalten werden.

Ist der Sender in ein Wachs, insbesondere in Paraffin, eingeschmolzen, so wird neben der Maximaltemperatur des

- 4 -

Senders, den phamokologischen Bedingungen besonders günstig Rechnung getragen.

Im folgenden wird die Erfindung anhand der Zeichnungen näher erläutert.

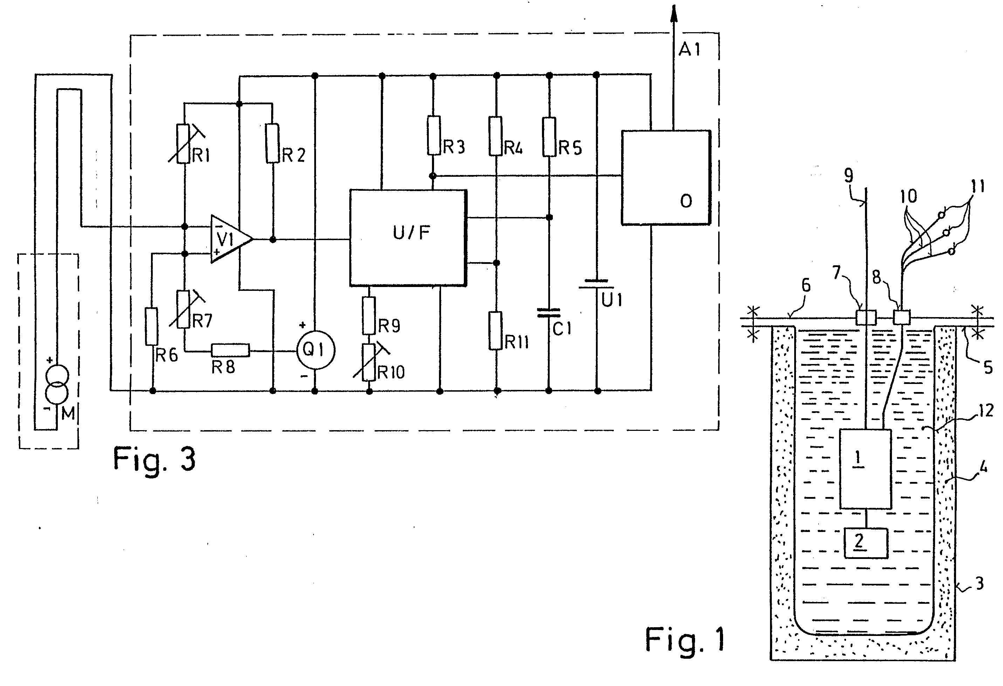

Es zeigen Fig. 1 einen Schnitt durch den Senderbehälter, Fig. 2 die Empfängerantenne und die Figuren 3 und 4 schematische Schaltungen des Senders bzw. Empfängers.

Der Sender 1 ist gemeinsam mit der Stromversorgung 2 in einem metallischen, doppelmanteligen Behälter 3 angeordnet. Der Hohlraum zwischen den beiden Mänteln des Behälters ist evakuiert und mit Kieselgur 4 gefüllt. Der Behälter weist einen Flansch 5 auf, auf welchem ein Deckel 6 befestigt ist. Dieser Deckel kann entweder genauso doppelmantelig ausgeführt sein, wie der restliche Behälter, oder z.B. aus einem niedrig wärmeleitenden Material aufgebaut sein. Der Deckel weist elektrisch isolierende Durchleitungen 7 und 8 auf, wobei durch eine Druchführung die Antenne 9 und durch die andere Durchführung die Verbindungsdrähte 10, in diesem Falle 3, zu den Meßsonden 11 geführt sind. Der Zwischenraum zwischen dem Behälter 3 und dem Sender 1 ist mit einem Paraffin 12, das einen Schmelzpunkt von 60°C aufweist, gefüllt. Ein derartiger Sender kann nun zwischen dem zu sterilisierenden Gut angeordnet werden, wobei die Meßfühler an verschiedenen Orten des Wagens leicht anordenbar sind. Als Meßfühler haben sich beispielsweise Thermoelemente und Widerstandsthermometer für die Temperaturmessung als geeignet erwiesen, wohingegen für Druckmessungen druckempfindliche Quarze und dgl. geeignet sind. Innerhalb des Behälters 3, insbesondere im Sender 1, kann auch ein Temperaturmeßfühler angeordnet werden, um die Funktionsfähigkeit des Senders jederzeit überprüfbar zu machen. Wie bekannt, weisen Transistoren maximale Betriebstemperatur auf, die unterhalb der Temperatur des Dampfes liegt.

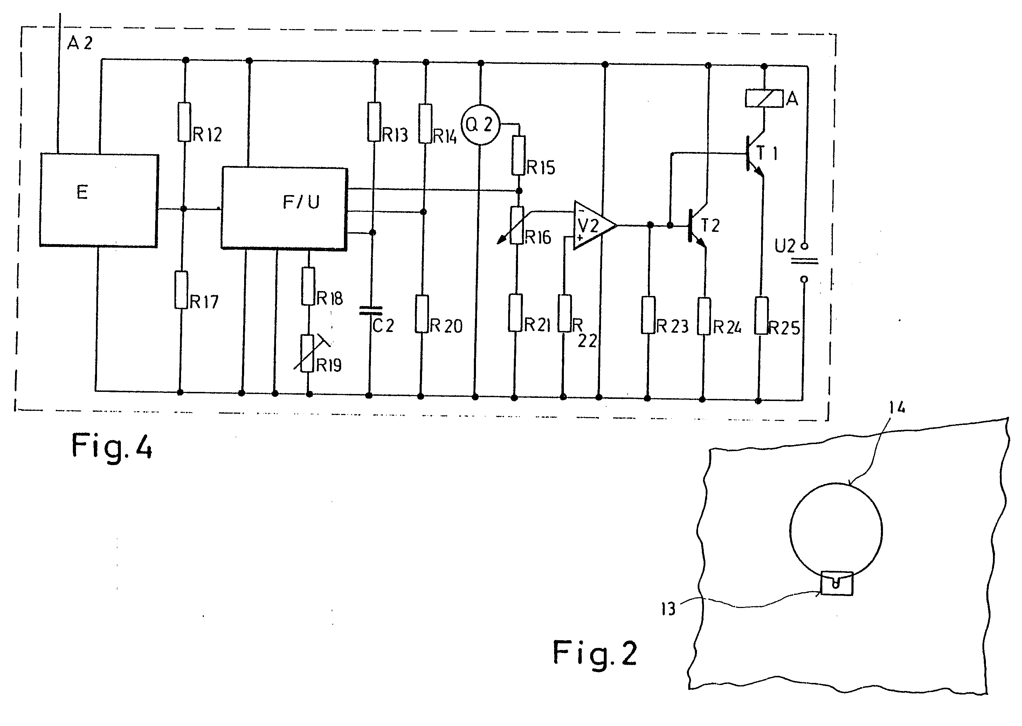

In Fig. 2 ist ein Teilausschnitt eines Wandbereiches des Dampfsterilisators dargestellt, wobei eine Durchführung 13 aus elektrisch isolierendem Material, z.B. Porzellan, für die Antenne 14 des Empfängers vorgesehen ist.

Die Steuerung des Sterilisators erfolgt nun derart, daß die einzelnen Meßsignale, z.B. die Temperaturmeßsignale, über den Sender mit Antenne 9 an die Antenne 14 weitergegeben werden. Um nicht für jeden einzelnen Meßfühler einen eigenen Sender vorzusehen, kann sowohl im Empfänger als auch im Sender ein sogenannter Multiplexer vorgesehen werden, wobei der Reihe nach die einzelnen Meßpunkte zu- bzw. abgeschaltet werden. Die Steuerung des Sterilisators erfolgt dann in an sich bekannter Art und Weise, wobei z.B. über ein Rechenprogramm die Temperatur im zu sterilisierenden Gut aufgrund des zeitlichen Temperaturverlaufs errechnet wird, und sowohl die Dampfzufuhr als auch die Drucksteuerung, das Be- und Entlüften über entsprechend gesteuerte Ventile erfolgt.

Bei dem in Fig. 3 schematisch dargestellten Blockschaltbild gehen die Signale von der Meßsonde M über den Widerstand $R_6$ in den Verstärker $V_1$ und werden dort verstärkt. Das in den Verstärker $V_1$ eingehende Signal wird dort verstärkt, wobei die Verstärkung über die veränderlichen Widerstände $R_1$ und $R_7$ eingestellt werden kann. Durch die Widerstände $R_2$ und $R_8$ sowie die Spannungskonstantquelle $Q_1$ wird die Referenzspannung für den Verstärker festgelegt. Im Spannungs-Frequenzumwandler wird das Signal aus dem

- 6 -

Verstärker $V_1$ in eine entsprechende Frequenz umgewandelt. Die Konkordanz zwischen Spannung und Frequenz
wird durch die Widerstände $R_9$ und $R_{10}$ sowie durch den
Kondensator $C_1$ festgelegt. Die Stromversorgung von U/F
erfolgt durch die Gleichstromspannungsquelle $U_1$ über
die Widerstände $R_3$, $R_4$, $R_5$ und $R_{11}$. Aus dem Spannungs-
Frequenzwandler geht das Signal zum Oszillator O, welcher
das Signal über die Antenne $A_1$ abstrahlt.

Das Signal, das von der Antenne $A_1$ abgestrahlt wird,
wird gemäß dem Blockschaltbild entsprechend Fig. 4 des
Empfängers E über die Antenne $A_2$ aufgenommen, verstärkt,
und dann demoduliert, wonach das Signal in den Frequenz-
Spannungsumwandler F/U geleitet wird. Für die Spannungsversorgung sowohl des Empfangteiles als auch des Frequenz-
Spannungsumwandlers über die Stromquelle $U_2$ sorgen die
Widerstände $R_{17}$, $R_{12}$, $R_{13}$, $R_{14}$ und $R_{20}$. Durch die Widerstände $R_{18}$, $R_{19}$ und $R_{13}$ sowie dem Kondensator $C_2$ wird
die Konkordanz zwischen Frequenz und Spannung regelbar
festgelegt. Die Referenzspannung wird durch die Spannungskonstantquelle $Q_2$ über den Widerstand $R_{15}$ dem Frequenzspannungswandler vorgegeben. Die Einstellung des Nullsignales für den Verstärker $V_2$ erfolgt über die Spannungskonstantquelle $Q_2$, die Widerstände $R_{15}$, $R_{21}$ und $R_{22}$,
wobei die Einstellung als solche über den veränderlichen Widerstand $R_{16}$ durchgeführt werden kann. Über
die Widerstände $R_{23}$ und $R_{24}$ wird der Arbeitspunkt des
Transistors $T_2$ festgelegt. Vom Ausgang des Transistors
$T_2$ kann bereits ein Signal entnommen werden, das über
einen entsprechenden Verstärker zur Steuerung von Ventilen od. dgl. dienen kann. Der Ausgang des Verstärkers
$V_2$ ist weiters mit dem Transistor $T_1$ verbunden, dessen
Arbeitspunkt über den Widerstand $R_{25}$ festgelegt wird.
Das ausgehende verstärkte Signal aus dem Transistor $T_1$

wird dem Relais A zugeführt, wobei dieses ebenfalls zur Schaltung von Elektroventilen und dgl. geeignet ist. Die Stromversorgung erfolgt über das Netz, in diesem Falle die Stromquelle $U_2$.

- 8 -

Patentansprüche :

1. Dampfsterilisator mit zumindest einem druckdicht abschließbaren Sterilisationsraum, in den das zu sterilisierende Gut, insbesondere Flüssigkeit enthaltende Behälter, z.B. Flaschen, Beutel od. dgl., einbringbar ist,
welcher Dampfzuleitungen und Gas- und/oder Flüssigkeitsableitungen aufweist, wobei eine Steuerung mit Temperaturmeßfühler im Sterilisationsraum vorgesehen ist, dadurch gekennzeichnet, daß der/die Meßfühler, insbesondere
Temperaturmeßfühler, mit einem Sender (1) zur drahtlosen
Übertragung von Signalen, mit Antenne (9) verbunden ist/sind,
der im Sterilisationsraum anordenbar ist und ein Empfänger zum Empfang der Signale außerhalb des Sterilisationsraumes angeordnet ist, welcher mit einer Antenne (14)
innerhalb desselben zur Weiterleitung der Signale an
die Steuerung verbunden ist.

2. Dampfsterilisator nach Anspruch 1, dadurch gekennzeichnet, daß die Antenne (14) des Empfängers gegenüber den
Wänden des Sterilisationsraumes elektrisch isoliert ist.

3. Dampfsterilisator nach Anspruch 1 oder 2, dadurch gekennzeichnet, daß mehrere Temperaturmeßfühler mit einem
Sender (1) verbunden sind.

4. Dampfsterilisator nach einem der Ansprüche 1, 2 oder
3, dadurch gekennzeichnet, daß der Sender (1) in einem, insbesondere metallischen, wasserdampfdichten Behälter (3) angeordnet ist.

5. Dampfsterilisator nach Anspruch 3, dadurch gekennzeichnet, daß der Sender (1) in einem evakuierten Behälter
angeordnet ist.

6. Dampfsterilisator nach Anspruch 5, dadurch gekennzeichnet, daß der Behälter (9) einen Doppelmantel aufweist,
welcher evakuiert ist und gegebenenfalls einen feinteiligen Füllstoff (4) enthält.

7. Dampfsterilisator nach Anspruch 4 bis 6, dadurch gekennzeichnet, daß der Sender (1) in einem Feststoff (12) mit einem
Schmelzpunkt zwischen 30 und $70^{O}$C, insbesondere zwischen
40 und $60^{O}$ eingeschmolzen ist.

8. Dampfsterilisator nach Anspruch 7, dadurch gekennzeichnet, daß der Sender (1) in ein Wachs, insbesondere
Paraffin, eingegossen ist.

Fig. 3

Fig. 1

0155933

Fig.2

Fig.4